Europäisches Patentamt

European Patent Office

Office européen des brevets

(11) Publication number: **0 410 066 A1**

# EUROPEAN PATENT APPLICATION

(21) Application number: 89810576.2

(22) Date of filing: 26.07.89

(51) Int. Cl.⁵: **G01N 33/68**, G01N 33/543, C07K 15/00

(43) Date of publication of application:
30.01.91 Bulletin 91/05

(84) Designated Contracting States:
**CH DE FR GB IT LI**

(71) Applicant: **SANDOZ AG**
**Lichtstrasse 35**
**CH-4002 Basel(CH)**

(72) Inventor: **Azria, Moise**
**Bundesplatz 6**
**CH-4054 Basel(CH)**

(54) **Improvements in or relating to organic compounds.**

㊗ The invention relates to an immunoassay for measuring bone metabolism turnover by detecting a bone biomarker in a blood sample and to a diagnosis kit providing the components for use in such an immunoassay.

EP 0 410 066 A1

## IMPROVEMENTS IN OR RELATING TO ORGANIC COMPOUNDS

The invention relates to an immunoassay for measuring bone metabolism turnover by detecting a bone biomarker in a blood sample and to a diagnosis kit providing the components for use in such an immunoassay.

Bone is a complex tissue and is constantly undergoing a complex process of renewal and remodeling involving many different factors and substances. By "bone turnover" is meant herein the relative rates of the competing processes of formation of new bone and resorption of old bone. Bone diseases such as osteoporosis, renal osteodystrophy and Paget's disease are usually correlated with an abnormal bone turnover, which should be detected as early as possible to ensure an effective treatment of the disease. This is particularly critical in the case of osteoporosis, a disease which affects almost 50% of postmenopausal women, since it is usually a silent disease. Typically a postmenopausal woman may have lost 20-30% of her bone mass and still be asymptomatic. Thus, there is a great need for a means of diagnosing the disease and of monitoring its course and its response to treatment, which should not involve resorting to bone biopsy.

Since some bone proteins also exist in a circulating form, measurements of circulating levels of such proteins should provide information on the status of the bone tissue. Products or by-products of bone formation or resorption should represent reliable indicators of bone remodeling.

Osteocalcin [also called bone GLA protein (BGP)] is a valuable biomarker for bone metabolism. Osteocalcin in fixed or circulating form is a 49 amino acid single-chain polypeptide with a molecular weight of 5,800 daltons which is secreted by osteoblasts and normally accumulated in the extracellular bone matrix as well as directly released into blood in minor quantities. Thus, the circulating form of osteocalcin is a naturally-occurring product arising from bone anabolism. On the other hand, fragments of osteocalcin which are also found in blood should be considered as degradation products resulting from bone resorption.

It has been proposed to measure osteocalcin in human serum using radioimmunoassays for the bovine protein, which cross-react with the human protein. Such assays involve the use of anti-bovine osteocalcin antisera (hereafter referred to as bovine antisera), which are known to recognize specifically the carboxy-terminal end of the molecule (Gundberg et al, Methods in Enzymology (1984) 107 : 516). Nowadays, several commercial kits are available (Cis-Oris; Seragen; Immunonuclear). However, normal human serum levels of osteocalcin range from 3 to 20 ng/ml, depending upon the antisera used and the laboratory procedures, which makes it extremely difficult to decide who is at risk and who is not. Further, although all of these kits measure an increase in serum osteocalcin concentration in a variety of patients with disorders characterized by high bone turnover, it is unclear how specific a particular assay is and how much of the molecule is actually measured. Indeed, an increase in serum osteocalcin concentration as measured by commercial kits is claimed to reflect a corresponding increase in release of osteocalcin from bone matrix during bone resorption. However, this may be hampered by the fact that the assays commercially available do not distinguish between the osteocalcin fragments and the whole molecule such as the circulating form which is a direct product of bone formation. Thus, there is still a great need for a standard assay method which would measure a single component rather than the whole molecule together with the fragments thereof.

An anti-human osteocalcin antiserum (hereafter referred to as human antiserum) has now been found which recognizes specifically the circulating form of osteocalcin to the exclusion of fragments thereof. Typically, the serum of patients with bone disorder e.g. osteoporotic patients has been tested with a commercially available kit (bovine antiserum) and by a new immunoassay developed with the human antiserum. Surprisingly, conflicting results were obtained i.e., while high osteocalcin levels were detected with the existing kit, very low levels were measured with the new immunoassay. This, in fact, reveals a decrease in serum concentration of the circulating form of osteocalcin in osteoporotic patients compared with normal adults. It is believed that such a decrease reflects a displacement of the balance between the amount of the form fixed in bone and the amount of the circulating form, in favour of the fixed form, rather than a decrease in osteocalcin synthesis by osteoblasts. Indeed, this displacement should be considered as the direct consequence of an accelerated degradation of osteocalcin in bone. Therefore, the amount of the circulating form of osteocalcin represents a specific index of bone turnover rather than of bone formation alone.

According to the invention, there is provided a diagnostic kit for specifically detecting in a human blood sample the osteocalcin molecule in a substantially complete form, to the exclusion of fragments thereof. The term "blood sample" refers herein to a sample of blood as such or to a sample of serum or plasma recovered from blood. By "osteocalcin in a substantially complete form" is meant hereinafter a molecule which has not been degraded into fragments such as those recognized by a bovine antiserum. This definition includes the circulating form of osteocalcin.

Advantageously, the kit of the invention ensures a detection level below 5 ng/ml osteocalcin, preferably below 3 ng/ml, more preferably below 1 ng/ml osteocalcin.

The kit of the invention comprises the components for use in an immunoassay preferably together with instructions for carrying out the test. A large number of immunological methods are available for detecting and titrating an antigen such as a protein in a sample of matter which may be a purified composition or a complex mixture. Among the most sensitive assays are the radioimmunoassays (RIAs) and the enzyme-linked-immunoadsorbent assays (ELISAs) both of these being performed in a competitive form. They may be liquid-phase or solid-phase immunoassays, the latter being preferred. By "solid-phase immunoassay" is understood an immunoassay in which one of the components is bound to a solid support which may be, for example, a gel or a microtitration plate.

The components provided within the kit include a reacting material and a tracer, the reacting material being preferably in a immobilized form, more preferably coated on the bottom of the wells of a microtitration plate. The reacting material may be purified osteocalcin, anti-osteocalcin antiserum, anti-osteocalcin immunoglobulins G (IgG) composition or anti-osteocalcin monoclonal antibody (MAb). It is preferred that the reacting material is purified osteocalcin provided in known quantities and in an unlabelled form.

The tracer provided within the kit is purified osteocalcin when the reacting material is an antiserum, an IgG composition or a MAb; whereas it is an antiserum, an IgG composition or a MAb, (all specific for osteocalcin) when the reacting material is purified osteocalcin, as is preferred. Further, the tracer is provided in a free and labelled form. The tracer may be labelled by radioisotopes such as $^{125}I$ or by covalently attaching to the tracer an enzyme which can react with a colourless substrate to give a coloured product. Alkaline phosphatase and p-nitrophenyl phosphate or horseradish peroxidase and O-phenylenediamine are examples of the enzyme and substrate respectively, the latter being preferred for use in the present invention.

When the tracer is coupled to an enzyme, then the kit further comprises a composition containing the substrate of said enzyme in addition to the tracer and the reacting material.

For use in radioimmunoassay, the tracer may be provided within the kit either in a labelled form or in an unlabelled form together wih a composition containing a radioisotope. The tracer is preferably provided in a liquid composition which preferably contains from 1 to 6% bovine serum albumin, more preferably from 2 to 5% most preferably 4% BSA. Alternatively, the tracer may be provided within the kit in solid form together with a liquid preparation to be used as a diluent of the tracer.

When the reacting material is purified osteocalcin in a free form i.e. not bound to a matrix, and the tracer a first MAb, then the kit may also comprise a second MAb specific for osteocalcin preferably linked to a support, more preferably to a microtitration plate, said first and second MAbs recognizing different epitopes on the osteocalcin molecule i.e. not competing with each other for the same binding site.

For use within the kit of the invention, osteocalcin is preferably human osteocalcin and the antiserum, IgG composition or MAb is preferably specific for human osteocalcin. An antiserum or an IgG composition thereof is specific for human osteocalcin as long as it is raised against human osteocalcin and its cross-reactivity with bovine osteocalcin is less than 20%. A MAb specific for human osteocalcin is a MAb raised against human osteocalcin. It is also preferred that the reacting material or the tracer be an IgG composition or a MAb rather than a crude antiserum.

It has also been found that the antiserum of the invention raised against human osteocalcin or an IgG composition thereof cross-reacts only slightly (less than 1%) with bovine osteocalcin. This suggests that the antiserum or the IgG composition thereof contains polyclonal antibodies which essentially recognize an epitope specific for the human osteocalcin.

For use in the present invention, the antiserum raised against human osteocalcin or an IgG composition thereof shows advantageously a cross-reactivity of less than 20%, preferably less than 10%, more preferably less than 1% with bovine osteocalcin. The percentage of cross-reactivity may be measured according to the following formula in which the osteocalcin concentrations are those necessary for displacing 50% of a tracer in an immunoassay:

$$\frac{[\text{human osteocalcin}]}{[\text{bovine osteocalcin}]} \times 100$$

In a preferred embodiment, a diagnostic test for specifically detecting and titrating the circulating form of osteocalcin in a blood sample may be performed as follows: To a polyvinyl chloride microtitration plate coated with purified human osteocalcin, are added aliquots of a dilution containing anti-human osteocalcin IgG's labelled with horseradish peroxidase. Preferably this dilution also contains 4% bovine serum albumin (BSA). With the serum recovered from the patient a range of dilutions is prepared, preferably in a solution containing 4% BSA. Aliquots of each dilution are applied to the wells. After washing, O-phenylenediamine is added. After a suitable reaction time, the excess of reagent is removed by washing and the plate is examined by spectrophotometry. The titre of the blood sample is determined with a standard curve which should be provided within the kit.

There is also provided a method for preparing an antiserum composition specific for the human osteocalcin molecule in a substantially complete form to the exclusion of fragments thereof, which comprises immunizing a non-human mammal with purified human osteocalcin and recovering the antiserum from the mammal.

According to another aspect of the invention, there is provided an antiserum composition which specifically reacts with the human osteocalcin molecule, in a substantially complete form, to the exclusion of fragments thereof.

The antiserum preferably comprises immunoglobulins which are essentially of the IgG isotype. Such an antiserum composition may be produced by removing the immunoglobulins of the IgA, IgGD, IgE and IgM isotypes from a crude antiserum by known methods.

The invention is further illustrated in the following examples with reference to the accompanying drawings.

Figure 1 shows the elution profile of human osteocalcin by gel filtration on Sephacryl S-200. Proteins are detected at 280 nm with an Uvikon spectrophotometer (810/820; kontron). Peak I corresponds to the dead volume. Peak II corresponds to the human osteocalcin as detected by immunoassay and peak III contains peptides of unknown specificity.

Figure 2 shows the elution profile of human osteocalcin by ion exchange chromatography on DEAE-Trisacryl. Osteocalcin is eluted under a NaCl gradient (X-X). Proteins (●.●) are detected at 280 nm.

Figure 3 shows the immunopurification of human osteocalcin on an immunoaffinity gel anti-bovine osteocalcin. Fractions are eluted with glycin buffer 200 mM pH 2.5 and analysed by ELISA (o-o). Proteins are detected at 280 nm (•-•)

Figure 4 shows the standard curve established in the ELISA assay with 2 ng human osteocalcin, coated per well using a tracer solution diluted at 1/800 in 4 % BSA-PB. This indicates the percentage of tracer which is not displaced per amount of free osteocalcin (ng/ml). B is the optical density measured in the absence of free

osteocalcin while $B_0$ is the optical density measured when adding a sample of free osteocalcin.

Example 1: Purification of human osteocalcin

**A) Preparation of the crude extract**

Bone diaphyses from healthy human thigh- and shinbones are freeze dried at -80°C in the form of small fragments. The bone fragments are powdered with a crusher in the presence of liquid nitrogen. Then the powder so obtained is washed with 5 volumes of Tris-HCl buffer (20 mM; pH 8) containing 4 antiproteases (Benzamidine-HCl 50 mM; phenylmethylsulphonyl fluoride (PMSF) 0.3 mM; 6-aminocaproic acid 100 mM and p-chloromercurybenzoic acid 1 mM) twice 2 hours at 4°C.

The powder is then suspended in 5 volumes 500 mM EDTA pH 6.2 containing the 4 antiproteases above mentioned at the same concentration. This mixture is stirred for 24 to 48 hours at 4°C and then centrifuged 5 min, 5000 xg at 4°C. The supernatant is recovered and dialysed against distilled water with a dialysis tube Spectra/POR 3 for one week at 4°C. The dialysis water is changed twice every 24 hours. The last dialysis is performed against ammonium carbonate buffer 50 mM pH 7.5. The preparation is frozen at -80°C, lyophilized and resuspended in 5 ml Tris-HCl buffer (20 mM; pH 8).

**B) Purification by gel filtration and ion-exchange chromatography**

This crude extract is further applied on a column of Sephacryl S-200 gel (Pharmacia) equilibrated with Tris-HCl buffer (20 mM; pH 8) and eluted with the same buffer at a flow rate of 17 ml/hour.

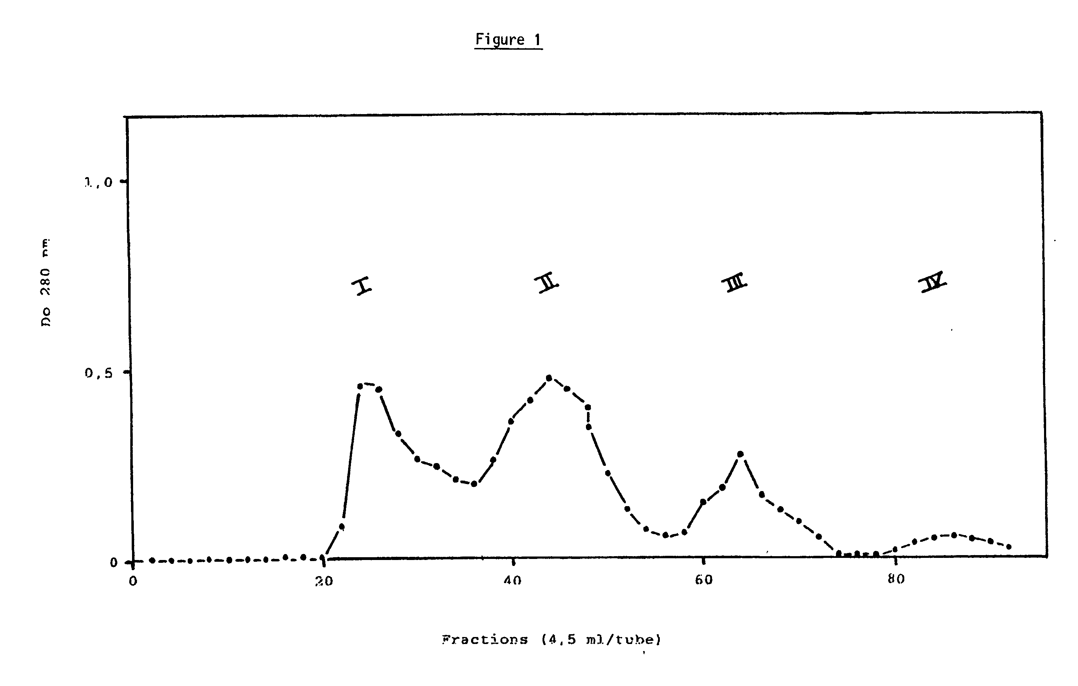

4.5 ml fractions are recovered and analysed by ELISA using an anti-bovine osteocalcin antiserum. The elution profile is to be seen on Figure 1. The human osteocalcin is detected in fractions 38-50.

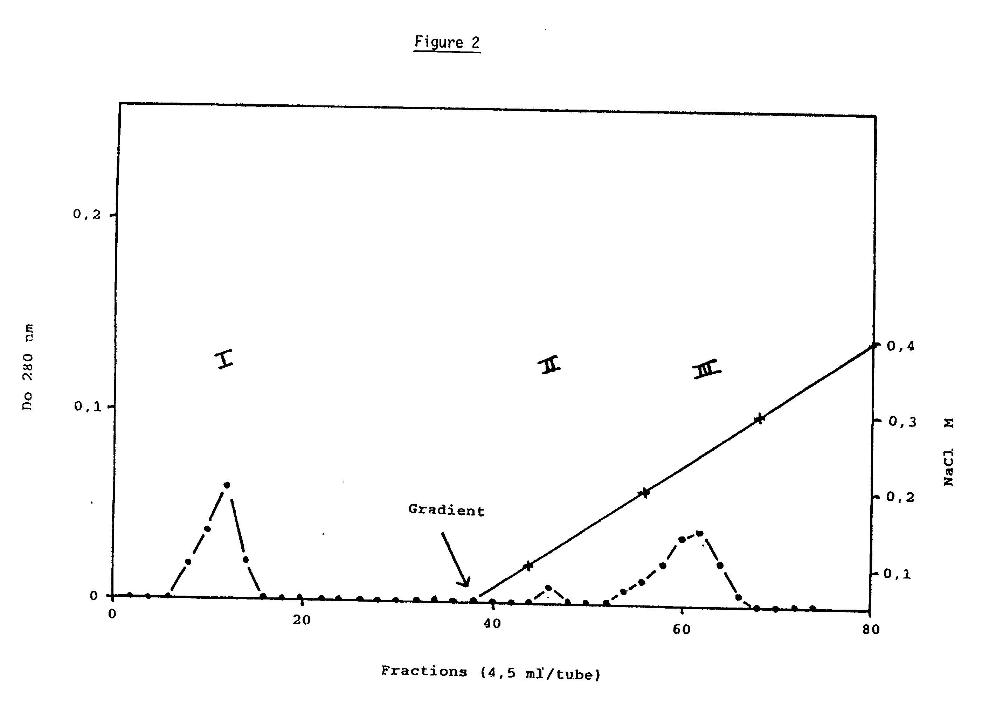

Osteocalcin-containing fractions (40 ml) are collected and applied on a column of ion-exchange chromatography gel (DEAE-Trisacryl M; IBF Biotechnics) equilibrated with Tris-HCl buffer (500 mM pH 8). Elution is performed under a NaCl gradient from 50 mM to 400 mM at a flow rate of 40 ml/hour. 4.5 ml fractions are recovered. As shown on Figure 2 human osteocalcin is eluted between 275 and 300 mM NaCl.

Osteocalcin-containing fractions are dialysed against ammonium carbonate buffer 50 mM pH 7.5 one night at 4°C, frozen at -80°C and lyophilized. For use, osteocalcin is resuspended in Tris-HCl buffer 100 mM pH 8.

## C) Purification by immunoaffinity

Alternatively, the crude extract may be purified by immunoaffinity.

The immunoaffinity gel is prepared as follows: To 1 g activated Sepharose-CNBr (Pharmacia) 3 mg anti-bovine osteocalcin antiserum are coupled in $NaHCO_3$ 100 mM pH 8.3; NaCl 500 mM. The preparation is incubated for 2 hours at room temperature. Ethanolamine 1 M is used for blocking the remaining active groups. Antiserum excess is removed by acidic and basic washings alternatively. The gel is mounted within a glass column (1.2 x 34 cm) and equilibrated with Tris-HCl 200 mM pH 8, NaCl 500 mM.

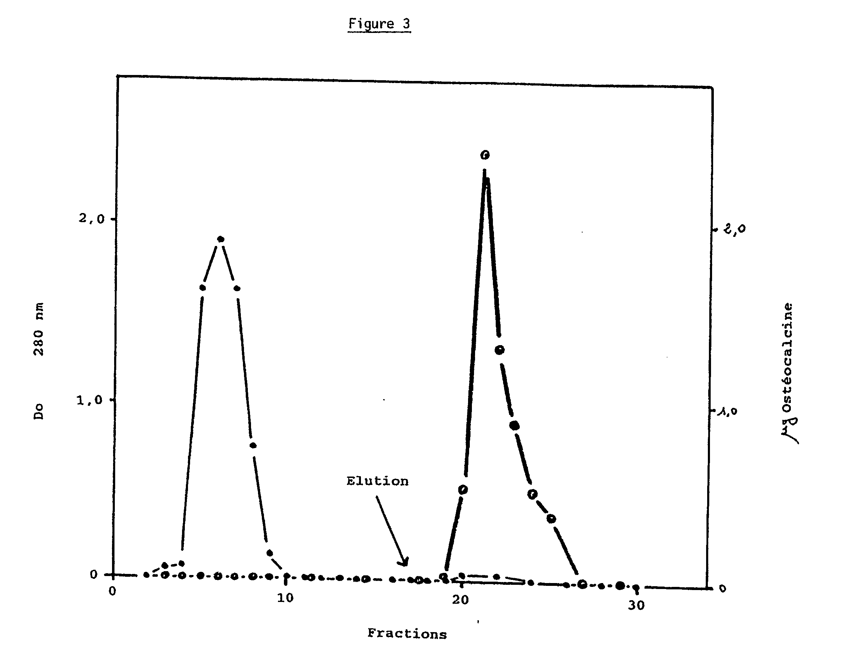

0.5-1 ml crude extract is applied on the column and left to stand for 1-2 hours at room temperature or 30 min at 37°C. The gel is then washed with Tris-HCl 20 mM pH 8; NaCl 500 mM. Elution is performed with a glycin buffer 200 mM pH 2.5. The elution profile is to be seen on Figure 3.

Example 2: Preparation of an antiserum

Fauve de Bourgogne rabbits are primarily immunized twice at 15 day interval with 50-100 µg human osteocalcin as purified in Example 1, in the presence of Freund adjuvant, (Gibco Lab.) by subcutaneous injection. One month later, the rabbits are boosted twice, at one month interval, with 50-100 µg human osteocalcin in the presence of 0.9% NaCl by intramuscular injection. 10 days after the last injection the antiserum (blood serum) is recovered and divided into two parts. One is kept as such at -20°C, the other one is purified with caprylic acid (Merck, ref. 193) by the method of Steinbuch et al (Arch. Biochem. Biophys. 134, 279-284, 1969) modified as follows: To 1 ml antiserum is added 2 ml acetate buffer 60 mM, pH 4 and 82 µl caprylic acid whilst stirring for 10 min. The solution is centrifuged 10,000 xg for 5 min. The supernatant is filtered on a 0.22 µg filter and dialysed against $Na_2CO_3/NaHCO_3$ 0.01 M pH 9.5 for 16 hours at 4°C. The dialysed solution containing IgG's is further kept at -20°C.

Example 3: The ELISA assay

### A) Preparation of the plate

Human osteocalcin as purified in Example 1 is coated on the bottom of wells of a microtitration plate (Nunc or Costar 3590) as follows:

Human osteocalcin is diluted at 20 ng/ml in Tris-HCl buffer 10 mM pH 8; NaCl 0.1 M. 100 µl of this solution (i.e. 2 ng osteocalcin) are applied per well with the exception of the first and second columns. The plate is incubated for 16 hours at 4°C and then washed with distilled water.

The wells are saturated with 150 µl of a 2 % bovine serum albumin solution in $NaH_2PO_4/Na_2HPO_4$ buffer 100 mM pH 7.2. The plate is incubated 30 min at 37°C and washed with distilled water.

### B) Preparation of the tracer

The anti-human osteocalcin IgG composition as prepared and purified in Example 2 is coupled to horseradish peroxidase by the Nakane method (J. Histochem. Cytochem. (1974) 22 : 1084) as follows: 5 ml peroxidase are diluted in 1 ml potassium phosphate buffer 0.1 M pH 6.8. Then 1 ml sodium bicarbonate 0.3M and 0.1 ml ethanol containing 2-4 dinitrofluorobenzene 0.1 g/l are added to the solution. The mixture is stirred for 2 hours at 20°C in the dark. Then 1 ml sodium periodate 80 mM is added. The mixture is further kept in the dark at 20°C whilst stirring. The reaction is stopped by adding 0.2 ml glycerol. The solution is further dialysed against $Na_2CO_3/NaHCO_3$ 10 mM pH 9.5 for 16 hours at 4°C.

The dialysed solution is mixed with 1 ml anti-human osteocalcin IgG solution 10 mg/ml as prepared in Example 2, stirred for 3 hours at 20°C in the dark, and dialysed against a potassium phosphate buffer 0.1 M pH 7.4 for 16 hours at 4°C. The final dialysed solution may be kept at -20°C.

### C) Dosage of the samples

All the dilutions (osteocalcin dilution series, samples and tracer) are performed with $NaH_2PO_4/Na_2HPO_4$ 100 mM pH 7.2, 4% BSA.

A standard dilution series from 1 to 100 ng/ml is prepared with human osteocalcin as prepared in Example 1. The tracer solution is prepared at a 1/800 dilution.

The wells are distributed on the plate as prepared in A) according to the following protocol:
column 1: 100 µl BSA-phosphate buffer
column 2: 50 µl BSA-phosphate buffer
50 µl tracer
column 3 to 11: 50 µl sample of free human osteocalcin
50 µl tracer

column 12: 50 $\mu$l -BSA-phosphate buffer
50 $\mu$l tracer.

The plate is incubated for 2 hours at 37° C and washed 4 times with distilled water. The plate is revealed by adding to each well 100 $\mu$l of the peroxidase substrate prepared as follows: To 10.5 ml citrate buffer pH 5.8, 30 mg O-phenylenediamine (OPD) is added and then 7.8 $\mu$l $H_2O_2$. The plate is incubated from 30 min at room temperature. The reaction is stopped by adding 100 $\mu$l/well sulphuric acid 1M. The plate is examined by spectrophotometry at 492 nm.

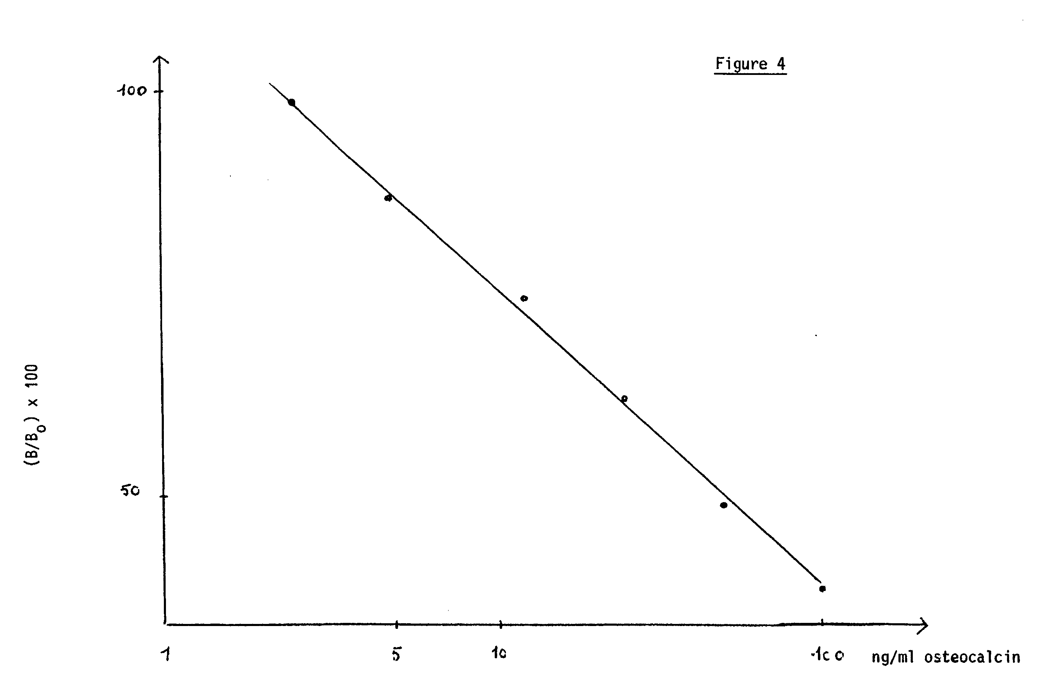

The standard curve obtained with human osteocalcin/anti-human osteocalcin antiserum is to be seen on Figure 4. Very good results are obtained within the range of linearity (2-100 ng/ml) and the limit of detection (sensitivity level: 2 ng/ml osteocalcin).

Example 4:

The sera of normal adults and osteoporotic patients are tested with the competitive ELISA of Example 3 and with a commercially available RIA assay (CIS-ORIS). Data obtained by ELISA (less than 3 ng/ml) reveal an apparent decrease in osteocalcin concentration in blood of osteoporotic patients while those obtained by RIA show an increase as compared with results usually obtained with normal adults.

**Claims**

1. A kit for specifically detecting in a human blood sample the presence of the osteocalcin molecule in a substantially complete form, to the exclusion of fragments thereof.

2. A kit according to claim 1 characterized by a sensitivity level in the order of 2 ng/ml.

3. A kit according to claim 1 or 2 comprising a reacting material and a tracer; said tracer being radio-labelled or linked to an enzyme.

4. A kit according to claim 3 in which the tracer is linked to an enzyme and which further comprises the substrate of said enzyme.

5. A kit according to claim 4 in which the enzyme is horseradish peroxidase and the substrate is O-phenylenediamine.

6. A kit according to any one of claims 3 to 5 in which the tracer is selected from human osteocalcin, an antiserum, an IgG composition thereof and a monoclonal antibody, said antiserum, said IgG composition and said monoclonal antibody being specific for human osteocalcin.

7. A kit according to claim 6 in which the tracer is an IgG composition.

8. A kit according to any one of claims 1 to 5 in which the reacting material is selected from purified human osteocalcin, an antiserum, an IgG composition thereof and a monoclonal antibody; said antiserum, said IgG composition and said monoclonal antibody being specific for human osteocalcin.

9. A kit according to claim 8 in which the reacting material is an IgG composition.

10. A kit according to any one of claims 3 to 9 in which the reacting material is in an immobilized form.

11. A kit according to any one of claims 3 to 8 in which the tracer is a first monoclonal antibody and which further comprises a second monoclonal antibody specific for the human osteocalcin, said first and second monoclonal antibodies recognizing different epitopes on the osteocalcin molecule.

12. A kit according to claim 11 in which the second antibody is in an immobilized form.

13. An antiserum composition which specifically reacts with the osteocalcin molecule, in a substantially complete form, to the exclusion of fragments thereof.

14. An antiserum composition according to claim 13, which is specific for human osteocalcin.

15. An antiserum composition according to claim 13 or 14 consisting essentially of immunoglobulins of the IgG isotype.

16. A method for preparing an antiserum composition specific for the osteocalcin molecule in a substantially complete form, to the exclusion of fragments thereof, which comprises immunizing a non-human mammal with purified human osteocalcin and recovering the antiserum from the mammal.

17. A method according to claim 16 which further comprises removing the immunoglobulins of the IgA, IgD, IgE and IgM isotypes from the recovered antiserum.

Figure 1

Fractions (4,5 ml/tube)

Figure 2

Figure 3

μg ostéocalcine

Fractions

Elution

Do 280 nm

Figure 4

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | JOURNAL OF IMMUNOLOGICAL METHODS, vol. 94, 1986, pages 19-24; H. TANAKA et al.: "An enzyme immunoassay for osteocalcin" * Whole document * | 1,3,4, 10,13, 15 | G 01 N 33/68 G 01 N 33/543 C 07 K 15/00 |
| X | METABOLISM, vol. 37, no. 9, September 1988, pages 872-877; A.K. TAYLOR et al.: "Development of a new radioimmunoassay for human osteocalcin: Evidence for a midmolecule epitope" * Whole document * | 1,3,6,7 ,13-16 | |
| A | PROC. NATL. ACAD. SCI. USA, vol. 77, no. 4, April 1980, pages 2234-2238; P.A. PRICE et al.: "Radioimmunoassay for the vitamin K-dependent protein of bone and its discovery in plasma" * Abstract * | 1-3 | |
| A | EP-A-0 095 873 (THE REGENTS OF THE UNIVERSITY OF CALIFORNIA) | | TECHNICAL FIELDS SEARCHED (Int. Cl.5) G 01 N |

The present search report has been drawn up for all claims

| Place of search | Date of completion of the search | Examiner |
|---|---|---|
| THE HAGUE | 13-03-1990 | HITCHEN C.E. |